(19) Europäisches Patentamt
European Patent Office
Office européen des brevets

(11) Numéro de publication : **0 462 883 B1**

(12) # FASCICULE DE BREVET EUROPEEN

(45) Date de publication du fascicule du brevet : **15.12.93 Bulletin 93/50**

(51) Int. Cl.$^5$ : **A61K 7/13**

(21) Numéro de dépôt : **91401614.2**

(22) Date de dépôt : **17.06.91**

(54) **Procédé de teinture des fibres kératiniques avec des composés indoliques, compositions et dispositifs.**

(30) Priorité : **21.06.90 FR 9007752**

(43) Date de publication de la demande : **27.12.91 Bulletin 91/52**

(45) Mention de la délivrance du brevet : **15.12.93 Bulletin 93/50**

(84) Etats contractants désignés : **AT BE CH DE DK ES FR GB GR IT LI NL SE**

(56) Documents cités :
**FR-A- 1 166 172**
**FR-A- 2 636 237**
**FR-A- 2 649 887**
**GB-A- 2 187 456**
**GB-A- 2 211 517**

(73) Titulaire : **L'OREAL**
**14, Rue Royale**
**F-75008 Paris (FR)**

(72) Inventeur : **Cotteret, Jean**
**15, Allée des Meunier**
**F-78480 Verneuil-sur-Seine (FR)**

(74) Mandataire : **Casalonga, Axel et al**
**BUREAU D.A. CASALONGA - JOSSE**
**Morassistrasse 8**
**D-80469 München (DE)**

**EP 0 462 883 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à un procédé de teinture des fibres kératiniques mettant en oeuvre des composés indoliques, ainsi qu'aux compositions et aux dispositifs de mise en oeuvre.

Les composés de la famille des indoles, en particulier le 5,6-dihydroxyindole ainsi que ses dérivés sont bien connus pour leur utilisation dans la teinture des fibres kératiniques et notamment des cheveux humains (i.e. GB-2 211 517 et FR-1 166 172).

La demanderesse a découvert qu'il était possible d'obtenir avec certains composés indoliques définis ci-après, des nuances variées à reflets ou naturelles de blond clair à foncé, des nuances chaudes telles que des nuances cuivrées ou acajou, en mettant en oeuvre ces composés indoliques dans un milieu neutre ou acide, l'application de cette composition étant suivie par l'application d'une composition oxydante alcaline.

Elle a découvert, par ailleurs, qu'en utilisant ces composés indoliques particuliers dans le procédé défini ci-dessus, il était possible d'obtenir des teintures résistant bien à la lumière et aux traitements chimiques, tels que la permanente.

Les colorations obtenues grâce à l'utilisation de ces composés indoliques particuliers, dans le cadre du procédé conforme à l'invention, présentent également l'avantage d'être uniformes même après plusieurs superpositions. Elles sont par ailleurs bien couvrantes.

L'invention a donc pour objet un nouveau procédé de teinture mettant en oeuvre certains dérivés indoliques particuliers dans un milieu cosmétiquement acceptable, à un pH neutre ou acide, l'application de ces dérivés dans les conditions de pH indiquées étant suivie par l'application d'une solution oxydante alcaline.

L'invention a également pour objet les compositions mises en oeuvre dans le cadre de ce procédé.

D'autres objets de l'invention apparaîtront à la lecture de la description et des exemples qui suivent.

Le procédé conforme à l'invention est essentiellement caractérisé par le fait que l'on applique, dans un premier temps, une composition tinctoriale renfermant dans un milieu cosmétiquement acceptable aqueux, ayant un pH inférieur ou égal à 7, au moins un dérivé indolique répondant à la formule :

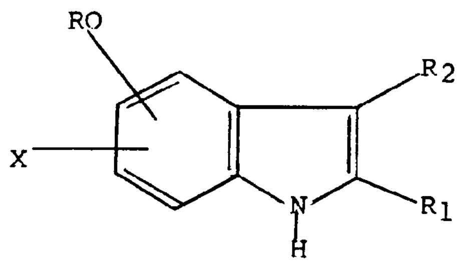

(I)

dans laquelle :

$R_1$ désigne hydrogène, alkyle ou COOH;

$R_2$ désigne hydrogène, alkyle;

R désigne hydrogène, un groupement $Si(CH_3)_3$, COalkyle;

X désigne hydrogène, OH, $OSi(CH_3)_3$; X en position 4 ou 5, pouvant également désigner Oalkyle, sous réserve

- que lorsque X est en position 5 et que OR désigne OH en position 4 ou 6, alors un seul des groupements $R_1$ et $R_2$ est différent d'hydrogène;
- que lorsque X désigne hydrogène, OR doit être en position 7 et $R_2$ doit désigner hydrogène;
- que lorsque R désigne hydrogène et X désigne OH, OR et X sont respectivement en position 5 et 6 et $R_1$ désigne COOH,

et les sels d'acides correspondants;

qu'après un temps de pose suivi d'un rinçage et d'un essorage, on applique dans un second temps une solution oxydante alcaline, cette application étant suivie d'un rinçage et d'un shampoing.

La demanderesse a constaté que ce procédé permettait d'obtenir des nuances variées à reflets ou des nuances naturelles de blonds clairs à foncés, de cuivre et d'acajou qui étaient uniformes après plusieurs superpositions et couvraient bien les cheveux.

Dans la formule (I), alkyle désigne de préférence un radical ayant 1 à 4 atomes de carbone.

Parmi les composés de formule (I) utilisables dans ce procédé conforme à l'invention, on peut citer le 7-hydroxy 2,3-diméthyl 4-méthoxyindole, le 7-hydroxy indole, le 5-acétoxy 6-hydroxyindole, le 6-hydroxy 2-méthyl 5-méthoxyindole, le 2-carboxy 5,6-dihydroxy indole, le 5,6-di(triméthylsilyloxy)indole, le 2-méthyl 4-hydroxy 5-éthoxyindole.

Les composés particulièrement préférés sont le 7-hydroxy 2,3-diméthyl 4-méthoxyindole, le 7-hydroxy indole, le 5-acétoxy 6-hydroxyindole et le 2-méthyl 4-hydroxy 5-éthoxyindole.

Selon une forme de réalisation préférée, ces composés peuvent être utilisés en mélange avec d'autres composés tels que le 5,6-dihydroxyindole, le 2-méthyl 5,6-dihydroxyindole ou le 6-hydroxyindole et son sel d'addition acide correspondant.

Le composé indolique de formule (I) utilisé conformément à l'invention, est présent de préférence dans la composition appliquée dans le premier temps dans des proportions comprises entre 0,02 et 5% et en particulier de 0,05 à 3% en poids par rapport au poids total de la composition.

Le milieu aqueux approprié pour la teinture est constitué par de l'eau et de préférence un mélange eau-solvant(s), le(s) solvant(s) étant choisi(s) parmi les solvants organiques tels que l'alcool éthylique, l'alcool propylique ou isopropylique, l'alcool tertio butylique, l'éthylèneglycol, les éthers monométhylique, monoéthylique ou monobutylique de l'éthylèneglycol, le propylèneglycol, les monométhyléthers du propylèneglycol et du dipropylèneglycol et le lactate de méthyle.

Les solvants préférés sont l'alcool éthylique et l'éther monobutylique de l'éthylèneglycol.

Dans le milieu eau-solvant(s), le(s) solvant(s) est (sont) présent(s) dans des concentrations comprises entre 0,5 et 75% en poids par rapport au poids total de la composition et en particulier entre 2 et 50% et de préférence entre 2 et 20% en poids.

Ce milieu peut également renfermer des agents tensio-actifs de préférence non-ioniques et des épaississants bien connus dans l'état de la technique, dans le domaine de la teinture des cheveux, tels que la gomme de guar, la gomme de xanthane, des polymères cellulosiques, dans des proportions comprises entre 0 et 3%.

Le pH peut être ajusté au moyen d'agents alcalinisants ou acidifiants habituellement utilisés dans la cosmétique capillaire.

La composition peut également renfermer des adjuvants habituellement utilisés en cosmétique, tels que des parfums ou des conservateurs.

En vue de nuancer les couleurs obtenues avec les dérivés indoliques de formule (I), on peut ajouter à la composition tinctoriale des colorants directs et/ou des précurseurs de colorants d'oxydation et/où des coupleurs et/ou des colorants d'oxydation rapides, bien connus dans le domaine de la coloration capillaire, comme par exemple dans l'ouvrage "HARRY'S COSMETICOLOGY" 7ème édition, édité par J.B.WILKINSON et R.J.MOORE, pages 521 à 545.

La composition oxydante est constituée de préférence par une solution aqueuse d'agent oxydant que l'on mélange au moment de l'emploi avec une solution aqueuse alcaline pouvant renfermer des solvants et des agents tensio-actifs du type défini ci-dessus.

L'agent oxydant est choisi de préférence parmi le peroxyde d'hydrogène, le peroxyde d'urée, les persels tels que les percarbonates, les perborates de métaux alcalins ou d'ammonium.

Les agents alcalins sont choisis parmi l'ammoniaque, les alcanolamines telles que le 2-amino 2-méthyl 1-propanol, la monoéthanolamine, la mono méthyléthanolamine ou la diméthyléthanolamine.

La solution aqueuse d'agent oxydant peut renfermer des solvants du type défini ci-dessus, des agents tensio-actifs ainsi que des cires auto-émulsionnables ou des alcools polyoxyéthylénés pour épaissir les solutions.

La proportion d'agent oxydant dans la composition appliquée dans le second temps, est comprise entre 1 et 15% en poids par rapport au poids total de la composition oxydante et de préférence entre 1 et 8% en poids.

La proportion d'agent oxydant dans la composition oxydante alcaline que l'on applique sur les cheveux est comprise entre 1 et 10% en poids par rapport au poids total de la composition et de préférence entre 1 et 5%.

Le pH de la composition oxydante est compris entre 8,5 et 12.

L'invention a également pour objet un agent de teinture des cheveux à deux composants.

Le premier composant (A) est constitué par une composition renfermant dans un milieu cosmétiquement acceptable un composé de formule (I) tel que défini ci-dessus et à un pH inférieur ou égal à 7; le second composant (B) est constitué par une solution aqueuse alcaline d'un agent oxydant telle que définie ci-dessus.

Cet agent de teinture peut être conditionné dans un dispositif à plusieurs compartiments ou kit de teinture, renfermant, dans des compartiments séparés, les composants (A) et (B).

Les exemples suivants sont destinés à illustrer l'invention sans pour autant présenter un caractère limitatif.

EXEMPLES 1 à 5

On procède à la teinture des cheveux en appliquant 60 g de la composition colorante ci-après.

On laisse agir 10 minutes, on rince abondamment à l'eau, on essore les cheveux puis on applique 75 g

de la composition oxydante qu'on laisse poser 10 minutes pour les exemples 1 et 5 et 20 minutes pour les exemples 2, 3 et 4.

Après rinçage et shampooing, on obtient la coloration indiquée en bas du tableau.

| en g | 1 | 2 | 3 | 4 | 5 |
|---|---|---|---|---|---|
| Composition colorante<br>7-hydroxy 2,3-diméthyl 4-méthoxy indole | | | | | 0,5 |
| 7-hydroxyindole | 1 | | | 0,05 | |
| 5-acétoxy 6-hydroxyindole | | 0,7 | | | |
| 2-méthyl 4-hydroxy 5-éthoxyindole | | | 1 | | |
| 2-méthyl 5,6-dihydroxyindole, HBr | | | | 1,5 | |
| 5,6- dihydroxyindole | 0,2 | | | | |
| Alcool éthylique | 10 | | 10 | 10 | 10 |
| Hydroxyéthylcellulose vendue par la Société HERCULES sous la dénomination NATROSOL 250 HHR | 1 | | 1 | 1 | 1 |
| Alkyléther de glycoside vendu à 60% de MA sous la dénomination TRITON CG 110 par la Société SEPPIC | 5 (MA) | | 5 (MA) | 5 (MA) | 5 (MA) |
| Lauryléthersulfate de sodium à 28% MA | | 4,2 (MA) | | | |
| Ether monobutylique de l'éthylène glycol | | 10 | | | |
| Triéthanolamine qs pH | | 5 | | | |
| pH spontané | 6,6 | | 6,5 | 6,8 | 6,5 |
| Eau qsp | 100 | 100 | 100 | 100 | 100 |

| en g | 1 | 2 | 3 | 4 | 5 |
|---|---|---|---|---|---|
| Composition oxydante : | 1/3A + 2/3 B | 1/3A + 2/3 B | 2/3A + 1/3 B | 1/3A + 2/3 B | 1/3A + 2/3 B |
| A) Nonylphénol oxyéthyléné à 4 moles d'oxyde d'éthylène | 26 | 26 | 26 | 26 | 26 |
| Nonylphénol oxyéthyléné à 9 moles d'oxyde d'éthylène | 24 | 24 | 24 | 24 | 24 |
| Ether monobutylique de l'éthylèneglycol | 13 | 13 | 13 | 13 | 13 |
| Propylèneglycol | 8 | 8 | 8 | 8 | 8 |
| Solution aqueuse d'ammoniaque à 20% | 19 | 19 | 19 | | 19 |
| Monoéthanolamine | | | | 8 | |
| Diéthanolamide d'acide oléique | 3,5 | 3,5 | 3,5 | 3,5 | 3,5 |
| Parfums, conservateurs, séquestrant | qs | qs | qs | qs | qs |
| Eau qsp | 100 | 100 | 100 | 100 | 100 |
| B) Eau oxygénée à 20 volumes | | | | | |
| Nuances obtenues : | châtain clair cendré | blond clair cendré doré | bleu violine puissant | blond foncé cuivré doré | acajou léger |

EXEMPLES 6 à 9

On procède à la teinture des cheveux en appliquant 60 g de la composition colorante ci-après.

On laisse agir 10 minutes, on rince abondamment à l'eau, on essore les cheveux puis on applique 75 g de la composition oxydante qu'on laisse poser 20 minutes pour les exemples 6 à 9.

Après rinçage et shampooing, on obtient la coloration indiquée en bas du tableau.

| Composition colorante : | 6 | 7 | 8 | 9 |
|---|---|---|---|---|
| 7-hydroxyindole | | | | 0,133 |
| 5,6-di-(triméthylsilyloxy)indole | | | 1 | |
| 2-méthyl 5-méthoxy 6-hydroxyindole | 0,5 | | | |
| 2-carboxy 5,6-dihydroxyindole | | 0,5 | | |
| 5,6-dihydroxyindole | | 0,5 | | |
| 6-hydroxyindole | | | | 0,133 |
| p-phénylènediamine | | | | 1,08 |
| p-aminophénol | | | | 0,436 |
| Alcool éthylique | 10 | 10 | 10 | 10 |
| Hydroxyéthylcellulose vendue par la Société HERCULES sous la dénomination NATROSOL 250 HHR | 1 | 1 | 1 | 1 |
| Alkyléther de glycoside vendu à 60% de MA sous la dénomination TRITON CG 100 par la Société SEPPIC | 5 (MA) | 5 (MA) | 5 (MA) | 5 (MA) |
| Acide citrique qs pH | | | | 6,7 |
| Triéthanolamine qs pH | | 6,8 | | |
| pH spontané | 6,6 | | 6,5 | |
| Eau qsp | 100 | 100 | 100 | 100 |

| en g | 6 | 7 | 8 | 9 |
|---|---|---|---|---|
| Composition oxydante : | 1/3A + 2/3B | 1/3A + 2/3B | 1/3A + 2/3B | 1/3A + 2/3B |
| A) Nonylphénol oxyéthyléné à 4 moles d'oxyde d'éthylène | 26 | 26 | 26 | 26 |
| Nonylphénol oxyéthyléné à 9 moles d'oxyde d'éthylène | 24 | 24 | 24 | 24 |
| Ether monobutylique de l'éthylèneglycol | 13 | 13 | 13 | 13 |
| Propylèneglycol | 8 | 8 | 8 | 8 |
| Solution aqueuse d'ammoniaque à 20% | 19 | 19 | 19 | 19 |
| Diéthanolamide d'acide oléique | 3,5 | 3,5 | 3,5 | 3,5 |
| Parfums, conservateurs, séquestrant | qs | qs | qs | qs |
| Eau qsp | 100 | 100 | 100 | 100 |
| B) Eau oxygénée à 20 volumes | | | | |
| Nuances obtenues : | Blond clair naturel doré | Châtain clair cendré doré | Blond très clair cendré | Blond cuivré doré |

**Revendications**

**Revendications pour les Etats contractants suivants : AT, BE, CH, DE, DK, FR, GB, GR, IT, LI, NL, SE**

1. Procédé de teinture des fibres kératiniques, en particulier des fibres kératiniques humaines telles que les cheveux, caractérisé par le fait que l'on applique sur ces fibres, dans un premier temps, une composition tinctoriale contenant dans un milieu cosmétiquement acceptable ayant un pH inférieur or égal à 7, au moins un dérivé indolique répondant à la formule :

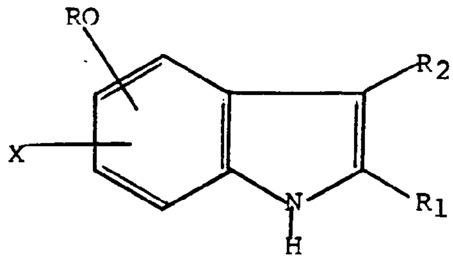

(I)

dans laquelle :

$R_1$ désigne hydrogène, alkyle or COOH;

$R_2$ désigne hydrogène ou alkyle;

R désigne hydrogène, $Si(CH_3)_3$, COalkyle;

X désigne hydrogène, OH, $OSi(CH_3)_3$; X en position 4 ou 5, pouvant également désigner le groupement Oalkyle, sous réserve

- que lorsque X est en position 5 avec les significations mentionnées ci-dessus, et que OR désigne OH en position 4 or 6, alors un seul des groupements $R_1$ et $R_2$ est différent d'hydrogène;
- que lorsque X désigne hydrogène, OR doit être en position 7 et $R_2$ doit désigner hydrogène;
- que lorsque R désigne hydrogène et X désigne OH, alors OR et X sont en position 5 et 6 et $R_1$ désigne COOH,

ainsi que les sels d'acides correspondants;

qu'après un temps de pose suivi d'un rinçage et d'un essorage on applique, dans un second temps, une solution oxydante alcaline dont l'application est suivie d'un rinçage et d'un shampooing.

2. Procédé selon la revendication 1, caractérisé par le fait que les dérivés indoliques sont choisis parmi le 7-hydroxy 2,3-diméthyl 4-méthoxyindole, le 7-hydroxyindole, le 5-acétoxy 6-hydroxyindole, le 6-hydroxy 2-méthyl 5-méthoxyindole, le 2-carboxy 5,6-dihydroxyindole, le 5,6-di(triméthylsilyloxy) indole, le 2-méthyl 4-hydroxy 5-éthoxyindole.

3. Procédé selon la revendication 1, caractérisé par le fait que les dérivés indoliques sont choisis parmi le 7-hydroxy 2,3-diméthyl 4-méthoxy indole, le 7-hydroxyindole, le 5-acétoxy 6-hydroxy indole et le 2-méthyl 4-hydroxy 5-éthoxyindole.

4. Procédé selon les revendications 1 à 3, caractérisé par le fait que le dérivé indolique de formule (I) est présent dans des proportions comprises entre 0,02 et 5% et de préférence entre 0,05 et 3% en poids.

5. Procédé selon l'une quelconque des revendications 1 à 4, caractérisé par le fait que le milieu de la composition tinctoriale est de l'eau ou un mélange eau-solvant(s), le(s) solvant(s) étant choisi(s) parmi l'alcool éthylique, l'alcool propylique ou isopropylique, l'alcool tertiobutylique, l'éthylène glycol, les éthers monométhylique, monoéthylique ou monobutylique de l'éthylèneglycol, le propylèneglycol, les monométhyléthers du propylèneglycol et du dipropylèneglycol et le lactate de méthyle.

6. Procédé selon l'une quelconque des revendications 1 à 5, caractérisé par le fait que la composition contient également un agent tensio-actif et un épaississant.

7. Procédé selon l'une quelconque des revendications 1 à 6, caractérisé par le fait que la composition tinctoriale renferme en plus d'autres composés indoliques.

8. Procédé selon la revendication 7, caractérisé par le fait que les autres composés indoliques sont choisis parmi le 5,6-dihydroxyindole, le 2-méthyl 5,6-dihydroxyindole, le 6-hydroxyindole et ses sels d'addition d'acides correspondants.

9. Procédé selon l'une quelconque des revendications 1 à 8, caractérisé par le fait que la composition tinctoriale renferme en plus d'autres colorants choisis parmi les colorants directs, les précurseurs de colorants d'oxydation, les coupleurs, les colorants d'oxydation rapides.

10. Procédé selon l'une quelconque des revendications 1 à 9, caractérisé par le fait que la solution oxydante contient un agent oxydant choisi parmi le peroxyde d'hydrogène, le peroxyde d'urée, les persels choisis

parmi les percarbonates, les perborates de métaux alcalins ou d'ammonium.

**11.** Procédé selon l'une quelconque des revendications 1 à 10, caractérisé par le fait que les agents alcalins utilisés dans la solution oxydante sont choisis parmi l'ammoniaque ou les alcanolamines.

**12.** Procédé selon l'une quelconque des revendications 1 à 11, caractérisé par le fait que la proportion d'agent oxydant dans la composition oxydante alcaline que l'on applique sur les cheveux, est comprise entre 1 et 10% en poids par rapport au poids total de la composition alcaline oxydante et de préférence entre 1 et 5%.

**13.** Agent de teinture des fibres kératiniques, en particulier des cheveux, caractérisé par le fait qu'il comporte deux composants : le premier composant (A) étant constitué par une composition tinctoriale renfermant dans un milieu aqueux cosmétiquement acceptable, un composé de formule (I) tel que défini dans l'une quelconque des revendications 1 à 9; le second composant (B) étant constitué par une solution oxydante alcaline préparée par mélange d'une solution aqueuse d'agent oxydant avec une solution alcaline telle que définies dans l'une quelconque des revendications 1 et 10 à 12.

**14.** Dispositif à plusieurs compartiments ou kit de teinture, caractérisé par le fait qu'il comporte au moins deux compartiments séparés, renfermant respectivement les composants (A) et (B) définis dans la revendication 13.

**Revendications pour l'Etat contractant suivant : ES**

**1.** Procédé de teinture des fibres kératiniques, en particulier des fibres kératiniques humaines telles que les cheveux, caractérisé par le fait que l'on applique sur ces fibres, dans un premier temps, une composition tinctoriale contenant dans un milieu cosmétiquement acceptable ayant un pH inférieur ou égal à 7, au moins un dérivé indolique répondant à la formule :

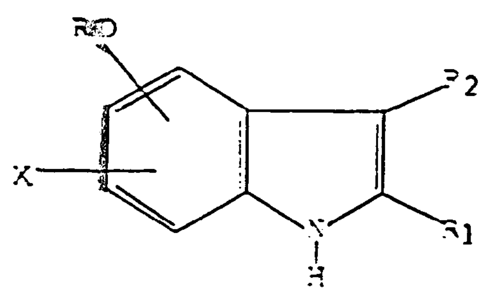

(I)

dans laquelle :

$R_1$ désigne hydrogène, alkyle ou COOH;

$R_2$ désigne hydrogène ou alkyle;

R désigne hydrogène, $Si(CH_3)_3$, COalkyle;

X désigne hydrogène, OH, $OSi(CH_3)_3$; X en position 4 ou 5, pouvant également désigner le groupement Oalkyle, sous réserve

- que lorsque X est en position 5 avec les significations mentionnées ci-dessus, et que OR désigne OH en position 4 ou 6 , alors un seul des groupements $R_1$ et $R_2$ est différent d'hydrogène;
- que lorsque X désigne hydrogène, OR doit être en position 7 et $R_2$ doit désigner hydrogène;
- que lorsque R désigne hydrogène et X désigne OH, alors OR et X sont en position 5 et 6 et $R_1$ désigne COOH,

ainsi que les sels d'acides correspondants;

qu'après un temps de pose suivi d'un rinçage et d'un essorage on applique, dans un second temps, une solution oxydante alcaline dont l'application est suivie d'un rinçage et d'un shampooing.

**2.** Procédé selon la revendication 1, caractérisé par le fait que les dérivés indoliques sont choisis parmi le 7-hydroxy 2,3-diméthyl 4-méthoxyindole, le 7-hydroxyindole, le 5-acétoxy 6-hydroxyindole, le 6-hydroxy 2-méthyl 5-méthoxyindole, le 2-carboxy 5,6-dihydroxyindole, le 5,6-di(triméthylsilyloxy) indole, le 2-méthyl 4-hydroxy 5-éthoxyindole.

3. Procédé selon la revendication 1, caractérisé par le fait que les dérivés indoliques sont choisis parmi le 7-hydroxy 2,3-diméthyl 4-méthoxy indole, le 7-hydroxyindole, le 5-acétoxy 6-hydroxy indole et le 2-méthyl 4-hydroxy 5-éthoxyindole.

4. Procédé selon les revendications 1 à 3, caractérisé par le fait que le dérivé indolique de formule (I) est présent dans des proportions comprises entre 0,02 et 5% et de préférence entre 0,05 et 3% en poids.

5. Procédé selon l'une quelconque des revendications 1 à 4, caractérisé par le fait que le milieu de la composition tinctoriale est de l'eau ou un mélange eau-solvant(s), le(s) solvant(s) étant choisi(s) parmi l'alcool éthylique, l'alcool propylique ou isopropylique, l'alcool tertiobutylique, l'éthylène glycol, les éthers monométhylique, monoéthylique ou monobutylique de l'éthylèneglycol, le propylèneglycol, les monométhyléthers du propylèneglycol et du dipropylèneglycol et le lactate de méthyle.

6. Procédé selon l'une quelconque des revendications 1 à 5, caractérisé par le fait que la composition contient également un agent tensio-actif et un épaississant.

7. Procédé selon l'une quelconque des revendications 1 à 6, caractérisé par le fait que la composition tinctoriale renferme en plus d'autres composés indoliques.

8. Procédé selon la revendication 7, caractérisé par le fait que les autres composés indoliques sont choisis parmi le 5,6-dihydroxyindole, le 2-méthyl 5,6-dihydroxyindole, le 6-hydroxyindole et ses sels d'addition d'acides correspondants.

9. Procédé selon l'une quelconque des revendications 1 à 8, caractérisé par le fait que la composition tinctoriale renferme en plus d'autres colorants choisis parmi les colorants directs, les précurseurs de colorants d'oxydation, les coupleurs, les colorants d'oxydation rapides.

10. Procédé selon l'une quelconque des revendications 1 à 9, caractérisé par le fait que la solution oxydante contient un agent oxydant choisi parmi le peroxyde d'hydrogène, le peroxyde d'urée, les persels choisis parmi les percarbonates, les perborates de metaux alcalins ou d'ammonium.

11. Procédé selon l'une quelconque des revendications 1 à 10, caractérisé par le fait que les agents alcalins utilisés dans la solution oxydante sont choisis parmi l'ammoniaque ou les alcanolamines.

12. Procédé selon l'une quelconque des revendications 1 à 11, caractérisé par le fait que la proportion d'agent oxydant dans la composition oxydante alcaline que l'on applique sur les cheveux, est comprise entre 1 et 10% en poids par rapport au poids total de la composition alcaline oxydante et de préférence entre 1 et 5%.

13. Agent de teinture des fibres kératiniques, en particulier des cheveux, caractérisé par le fait qu'il comporte deux composants : le premier composant (A) étant constitué par une composition tinctoriale renfermant dans un milieu aqueux cosmétiquement acceptable, un composé de formule (I) tel que défini dans l'une quelconque des revendications 1 à 9 ; le second composant (B) étant constitué par une solution oxydante alcaline préparée par mélange d'une solution aqueuse d'agent oxydant avec une solution alcaline telle que définies dans l'une quelconque des revendications 1 et 10 à 12.

14. Dispositif à plusieurs compartiments ou kit de teinture, caractérisé par le fait qu'il comporte au moins deux compartiments séparés, renfermant respectivement les composants (A) et (B) définis dans la revendication 13.

15. Procédé de préparation d'un agent de teinture des fibres kératiniques, en particulier des cheveux, caractérisé en ce que l'on incorpore, dans un milieu aqueux cosmétiquement acceptable tel que défini dans l'une des revendications 1 et 5 à 9, un composé de formule (I) tel que défini dans l'une quelconque des revendications 1 à 4, pour obtenir le premier composant (A) et en ce qu'on mélange une solution aqueuse d'agent oxydant avec une solution aqueuse alcaline pour obtenir une solution oxydante alcaline telle que définie dans l'une quelconque des revendications 1 et 10 à 12, et constituant le composant (B).

## Claims

### Claims for the following Contracting States : AT, BE, CH, DE, DK, FR, GB, GR, IT, LI, NL, SE

1. Method for dyeing keratinous fibres, in particular human keratinous fibres such as hair, characterised in that a tinctorial composition containing, in a cosmetically acceptable medium having a pH of less than or equal to 7, at least one indole derivative corresponding to the formula:

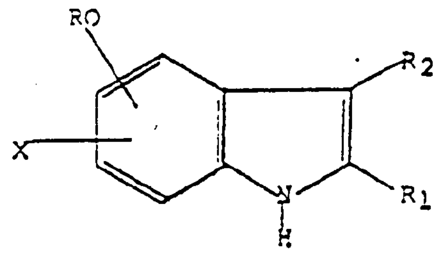

(I)

in which:

$R_1$ denotes hydrogen, alkyl or COOH;
$R_2$ denotes hydrogen or alkyl;
R denotes hydrogen, $Si(CH_3)_3$, or COalkyl; and
X denotes hydrogen, OH, or $OSi(CH_3)_3$; it being possible for X in the 4- or 5-position also to denote the Oalkyl group, on condition

- that when X is in the 5-position with the meanings mentioned above and OR denotes OH in the 4- or 6-position, only one of the groups $R_1$ and $R_2$ then differs from hydrogen;
- that when X denotes hydrogen, OR must be in the 7-position and $R_2$ must denote hydrogen; and
- that when R denotes hydrogen and X denotes OH, OR and X are then in the 5- and the 6-position and $R_1$ denotes COOH,

and the corresponding acid salts,

is applied to these fibres in a first step and in that, after a dwell time followed by rinsing and rubbing dry, an alkaline oxidising solution is applied in a second step, the application of which is followed by rinsing and shampooing.

2. Method according to Claim 1, characterised in that the indole derivatives are chosen from 7-hydroxy-2,3-dimethyl-4-methoxyindole, 7-hydroxyindole, 5-acetoxy-6-hydroxyindole, 6-hydroxy-2-methyl-5-methoxyindole, 2-carboxy-5,6-dihydroxyindole, 5,6-di(thirmethyl silyoxylindole and 2-methyl-4-hydroxy-5-ethoxyindole.

3. Method according to Claim 1, characterised in that the indole derivatives are chosen from 7-hydroxy-2,3-dimethyl-4-methoxyindole, 7-hydroxyindole,5-acetoxy-6-hydroxyindole and 2-methyl-4-hydroxy-5-ethoxyindole.

4. Method according to Claims 1 to 3, characterised in that the indole derivative of formula (I) is present in proportions of between 0.02 and 5% and preferably between 0.05 and 3% by weight.

5. Method according to any one of Claims 1 to 4, characterised in that the medium of the tinctorial composition is water or a water-solvent(s) mixture, the solvent(s) being chosen from ethyl alcohol, propyl or isopropyl alcohol, tert-butyl alcohol, ethylene glycol, ethylene glycol monomethyl, monoethyl or monobutyl ethers, propylene glycol, propylene glycol and dipropylene glycol monomethyl ethers and methyl lactate.

6. Method according to any one of Claims 1 to 5, characterised in that the composition also contains a surface-active agent and a thickener.

7. Method according to any one of Claims 1 to 6, characterised in that the tinctorial composition also contains other indole compounds.

8. Method according to Claim 7, characterised in that the other indole compounds are chosen from 5,6-dihydroxyindole, 2-methyl-5,6-dihydroxyindole or 6-hydroxyindole and its corresponding acid addition salts.

9. Method according to any one of Claims 1 to 8, characterised in that the tinctorial composition also contains other dyes chosen from direct dyes, precursors of oxidation dyes, couplers and rapid oxidation dyes.

10. Method according to any one of Claims 1 to 9, characterised in that the oxidising solution contains an oxidising agent chosen from hydrogen peroxide, urea peroxide and persalts chosen from alkali metal or ammonium percarbonates and perborates.

11. Method according to any one of Claims 1 to 10, characterised in that the alkaline agents used in the oxidising solution are chosen from ammonia or alkanolamines.

12. Method according to any one of Claims 1 to 11, characterised in that the proportion of oxidising agent in the akaline oxidising composition which is applied to the hair is between 1 and 10 % by weight relative to the total weight of the oxidising alkaline composition, and preferably between 1 and 5 %.

13. Agent for dyeing keratinous fibres, in particular hair, characterised in that it comprises two components : the first component (A) consisting of a tinctorial composition containing, in a cosmetically acceptable aqueous medium, a compound of formula (I) as defined in any one of Claims 1 to 9 and the second component (B) consisting of an alkaline oxidising solution prepared by mixing of an aqueous solution of oxidising agent with an alkaline aqueous solution as defined in any one of Claims 1 and 10 to 12.

14. Multi-compartment device or dyeing kit , characterised in that it comprises at least two separate compartments containing, respectively, the components (A) and (B) defined in Claim 13.

**Claims for the following Contracting State : ES**

1. Method for dyeing keratinous fibres, in particular human keratinous fibres such as hair, characterised in that a tinctorial composition containing, in a cosmetically acceptable medium having a pH of less than or equal to 7, at least one indole derivative corresponding to the formula:

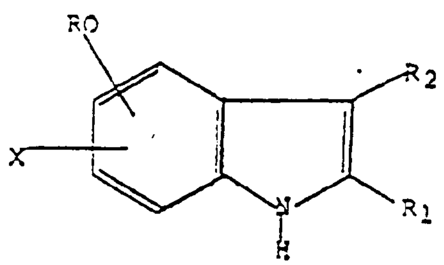

(I)

in which:

$R_1$ denotes hydrogen, alkyl or COOH;

$R_2$ denotes hydrogen or alkyl;

R denotes hydrogen, $Si(CH_3)_3$, or COalkyl; and

X denotes hydrogen, OH, or $OSi(CH_3)_3$; it being possible for X in the 4- or 5-position also to denote the Oalkyl group, on condition

- that when X is in the 5-position with the meanings mentioned above and OR denotes OH in the 4- or 6-position, only one of the groups $R_1$ and $R_2$ then differs from hydrogen;
- that when X denotes hydrogen, OR must be in the 7-position and $R_2$ must denote hydrogen; and
- that when R denotes hydrogen and X denotes OH, OR and X are then in the 5- and the 6-position and $R_1$ denotes COOH,

and the corresponding acid salts,

is applied to these fibres in a first step and in that, after a dwell time followed by rinsing and rubbing dry, an alkaline oxidising solution is applied in a second step, the application of which is followed by rinsing and shampooing.

2. Method according to Claim 1, characterised in that the indole derivatives are chosen from 7-hydroxy-2,3-dimethyl-4-methoxyindole, 7-hydroxyindole,5-acetoxy-6-hydroxyindole, 6-hydroxy-2-methyl-5-methoxyindole, 2-carboxy-5,6-dihydroxyindole, 5,6-di(thirmethyl silyoxylindole and 2-methyl-4-hydroxy-5-ethoxyindole.

3. Method according to Claim 1, characterised in that the indole derivatives are chosen from 7-hydroxy-2,3-dimethyl-4-methoxyindole, 7-hydroxyindole, 5-acetoxy-6-hydroxyindole and 2-methyl-4-hydroxy-5-ethoxyindole.

4. Method according to Claims 1 to 3, characterised in that the indole derivative of formula (I) is present in proportions of between 0.02 and 5% and preferably between 0.05 and 3% by weight.

5. Method according to any one of Claims 1 to 4, characterised in that the medium of the tinctorial composition is water or a water-solvent(s) mixture, the solvent(s) being chosen from ethyl alcohol, propyl or isopropyl alcohol, tert-butyl alcohol, ethylene glycol, ethylene glycol monomethyl, monoethyl or monobutyl ethers, propylene glycol, propylene glycol and dipropylene glycol monomethyl ethers and methyl lactate.

6. Method according to any one of Claims 1 to 5, characterised in that the composition also contains a surface-active agent and a thickener.

7. Method according to any one of Claims 1 to 6, characterised in that the tinctorial composition also contains other indole compounds.

8. Method according to Claim 7, characterised in that the other indole compounds are chosen from 5,6-dihydroxyindole, 2-methyl-5,6-dihydroxyindole or 6-hydroxyindole and its corresponding acid addition salts.

9. Method according to any one of Claims 1 to 8, characterised in that the tinctorial composition also contains other dyes chosen from direct dyes, precursors of oxidation dyes, couplers and rapid oxidation dyes.

10. Method according to any one of Claims 1 to 9, characterised in that the oxidising solution contains an oxidising agent chosen from hydrogen peroxide, urea peroxide and persalts chosen from alkali metal or ammonium percarbonates and perborates.

11. Method according to any one of Claims 1 to 10, characterised in that the alkaline agents used in the oxidising solution are chosen from ammonia or alkanolamines.

12. Method according to any one of Claims 1 to 11, characterised in that the proportion of oxidising agent in the akaline oxidising composition which is applied to the hair is between 1 and 10 % by weight relative to the total weight of the oxidising alkaline composition, and preferably between 1 and 5 %.

13. Agent for dyeing keratinous fibres, in particular hair, characterised in that it comprises two components : the first component (A) consisting of a tinctorial composition containing, in a cosmetically acceptable aqueous medium, a compound of formula (I) as defined in any one of Claims 1 to 9 and the second component (B) consisting of an alkaline oxidising solution prepared by mixing of an aqueous solution of oxidising agent with an alkaline aqueous solution as defined in any one of Claims 1 and 10 to 12.

14. Multi-compartment device or dyeing kit , characterised in that it comprises at least two separate compartments containing, respectively, the components (A) and (B) defined in Claim 13.

15. Process for preparing an agent for dyeing keratinous fibres, in particular hair, characterised in that a compound of formula (I) as defined in any one of Claims 1 to 4 is incorporated in a cosmetically acceptable aqueous medium as defined in any one of Claims 1 and 5 to 9 in order to obtain a first component (A), and in that an aqueous solution of oxidising agent is mixed with an alkaline aqueous solution in order to obtain an alkaline oxidising solution as defined in any one of Claims 1 and 10 to 12 which constitutes the component (B).

## Patentansprüche

### Patentansprüche für folgende Vertragsstaaten : AT, BE, CH, DE, DK, FR, GB, GR, IT, LI, NL, SE

1. Verfahren zum Färben von Keratinfasern, insbesondere menschlichen Keratinfasern wie den Haaren, dadurch gekennzeichnet, daß man auf diese Fasern in einem ersten Zeitabschnitt eine Färbungszusammensetzung aufbringt, welche in einem kosmetisch annehmbaren Milieu, das einen pH unter oder gleich 7 hat, wenigstens ein der folgenden Formel entsprechendes Indolderivat:

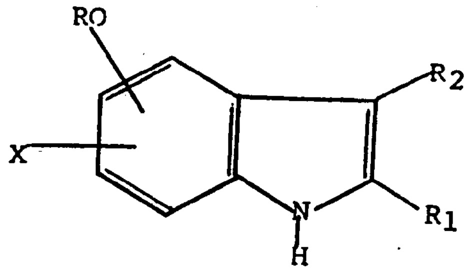

(I)

worin:

$R_1$ Wasserstoff, Alkyl oder COOH bedeutet;

$R_2$ Wasserstoff oder Alkyl bedeutet;

R Wasserstoff, $Si(CH_3)_3$, COalkyl bedeutet;

X Wasserstoff, OH, $OSi(CH_3)_3$ bedeutet, wobei X in Stellung 4 oder 5 ebenfalls die Gruppierung Oalkyl bedeuten kann, mit der Maßgabe,

- daß, falls X in Stellung 5 mit den oben angegebenen Bedeutungen steht, und falls OR in Stellung 4 oder 6 OH bedeutet, dann nur eine der Gruppierungen $R_1$ und $R_2$ von Wasserstoff verschieden ist;
- daß, falls X Wasserstoff bedeutet, OR in Stellung 7 sein muß und $R_2$ Wasserstoff bedeuten muß;
- daß, falls R Wasserstoff bedeutet und X OH bedeutet, dann OR und X in Stellung 5 und 6 sind, und $R_1$ COOH bedeutet,

oder die entsprechenden Säuresalze enthält;

daß nach einer Einwirkzeit, gefolgt von einem Spülen und einem Trocknen, man, in einem zweiten Zeitabschnitt, eine oxidierende, alkalische Lösung aufbringt, deren Auftrag von einem Spülen und einer Shampoobehandlung gefolgt ist.

2. Verfahren nach Anspruch 1, dadurch gekennzeichnet, daß die Indolderivate ausgewählt werden unter 7-Hydroxy-2,3-dimethyl-4-methoxyindol, 7-Hydroxyindol, 5-Acetoxy-6-hydroxyindol, 6-Hydroxy-2-methyl-5-methoxyindol, 2-Carboxy-5,6-dihydroxyindol, 5,6-Di(trimethylsilyloxy)indol, 2-Methyl-4-hydroxy-5-ethoxyindol.

3. Verfahren nach Anspruch 1, dadurch gekennzeichnet, daß die Indolderivate ausgewählt werden unter 7-Hydroxy-2,3-dimethyl-4-methoxyindol, 7-Hydroxyindol, 5-Acetoxy-6-hydroxyindol und 2-Methyl-4-hydroxy-5-ethoxyindol.

4. Verfahren nach den Ansprüchen 1 bis 3, dadurch gekennzeichnet, daß das Indolderivat der Formel (I) in zwischen 0,02 und 5 Gew.-% und vorzugsweise zwischen 0,05 und 3 Gew.-% liegenden Anteilen vorhanden ist.

5. Verfahren nach einem der Ansprüche 1 bis 4, dadurch gekennzeichnet, daß das Milieu der Färbungszusammensetzung Wasser oder eine Mischung Wasser-Lösungsmittel ist, wobei das/die Lösungsmittel ausgewählt wird/werden unter Ethylalkohol, Propyl- oder Isopropylalkohol, tert.-Butylalkohol, Ethylenglykol, Monomethyl-, Monoethyl- oder Monobutylethern von Ethylenglykol, Propylenglykol, Monomethylethern von Propylenglykol und Dipropylenglykol und Methyllactat.

6. Verfahren nach einem der Ansprüche 1 bis 5, dadurch gekennzeichnet, daß die Zusammensetzung ebenfalls ein grenzflächenaktives Mittel und ein Verdickungsmittel enthält.

7. Verfahren nach einem der Ansprüche 1 bis 6, dadurch gekennzeichnet, daß die Färbungszusammensetzung zusätzlich andere Indolverbinndungen enthält.

8. Verfahren nach Anspruch 7, dadurch gekennzeichnet, daß die anderen Indolverbindungen ausgewählt werden unter 5,6-Di-hydroxyindol, 2-Methyl-5,6-dihydroxyindol, 6-Hydroxyindol und ihren entsprechenden Säureadditionssalzen.

9. Verfahren nach einem der Ansprüche 1 bis 8, dadurch gekennzeichnet, daß die Färbungszusammensetzung zusätzlich andere Farbstoffe, ausgewählt unter Direktfarbstoffen, Vorläufern von Oxidationsfarbstoffen, Kupplern, schnellen Oxidationsfarbstoffen, einschließt.

**10.** Verfahren nach einem der Ansprüche 1 bis 9, dadurch gekennzeichnet, daß die oxidierende Lösung ein Oxidationsmittel, ausgewählt unter Wasserstoffperoxid, Harnstoffperoxid, den aus Perkarbonaten, Perboraten von Alkalimetallen oder Ammonium ausgewählten Persalzen, enthält.

**11.** Verfahren nach einem der Ansprüche 1 bis 10, dadurch gekennzeichnet, daß die in der oxidierenden Lösung verwendeten alkalischen Mittel unter Ammoniak oder Alkanolaminen ausgewählt werden.

**12.** Verfahren nach einem der Ansprüche 1 bis 11, dadurch gekennzeichnet, daß der Anteil des Oxidationsmittels in der alkalischen, oxidierenden Zusammensetzung, welche man auf die Haare aufbringt, zwischen 1 und 10 Gew.-%, bezogen auf das Gesamtgewicht der oxidierenden, alkalischen Zusammensetzung, vorzugsweise zwischen 1 und 5 %, beträgt.

**13.** Färbungsmittel für Keratinfasern, insbesondere Haare, dadurch gekennzeichnet, daß es zwei Bestandteile umfaßt, wobei der erste Bestandteil (A) durch eine Färbungszusammensetzung gebildet wird, die in einem kosmetisch annehmbaren, wässrigen Milieu eine Verbindung der Formel (I), wie in einem der Ansprüche 1 bis 9 definiert, umfaßt, und der zweite Bestandteil (B) durch eine oxidierende, alkalische Lösung gebildet wird, die durch Mischen einer wässrigen Lösung eines Oxidationsmittels mit einer alkalischen Lösung, wie in einem der Ansprüche 1 und 10 bis 12 definiert, hergestellt worden ist.

**14.** Vorrichtung mit mehreren Abteilungen oder Färbungskit, dadurch gekennzeichnet, daß sie/es wenigstens zwei getrennte Abteilungen umfaßt, welche jeweils die in Anspruch 13 definierten Bestandteile (A) und (B), einschließen.

**Patentansprüche für folgenden Vertragsstaat : ES**

**1.** Verfahren zum Färben von Keratinfasern, insbesondere menschlichen Keratinfasern wie den Haaren, dadurch gekennzeichnet, daß man auf diese Fasern in einem ersten Zeitabschnitt eine Färbungszusammensetzung aufbringt, welche in einem kosmetisch annehmbaren Milieu, das einen pH unter oder gleich 7 hat, wenigstens ein der folgenden Formel entsprechendes Indolderivat:

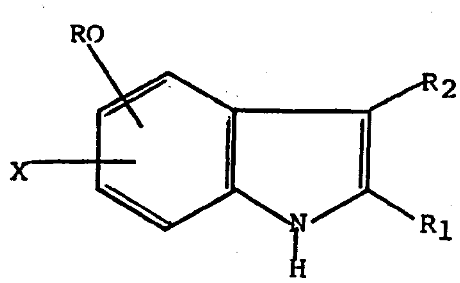

(I)

worin:

$R_1$ Wasserstoff, Alkyl oder COOH bedeutet;
$R_2$ Wasserstoff oder Alkyl bedeutet;
R Wasserstoff, $Si(CH_3)_3$, COalkyl bedeutet;
X Wasserstoff, OH, $OSi(CH_3)_3$ bedeutet, wobei X in Stellung 4 oder 5 ebenfalls die Gruppierung Oalkyl bedeuten kann, mit der Maßgabe,

- daß, falls X in Stellung 5 mit den oben angegebenen Bedeutungen steht, und falls OR in Stellung 4 oder 6 OH bedeutet, dann nur eine der Gruppierungen $R_1$ und $R_2$ von Wasserstoff verschieden ist;
- daß, falls X Wasserstoff bedeutet, OR in Stellung 7 sein muß und $R_2$ Wasserstoff bedeuten muß;
- daß, falls R Wasserstoff bedeutet und X OH bedeutet, dann OR und X in Stellung 5 und 6 sind, und $R_1$ COOH bedeutet,

oder die entsprechenden Säuresalze enthält;
daß nach einer Einwirkzeit, gefolgt von einem Spülen und einem Trocknen, man, in einem zweiten Zeitabschnitt, eine oxidierende, alkalische Lösung aufbringt, deren Auftrag von einem Spülen und einer Shampoobehandlung gefolgt ist.

**2.** Verfahren nach Anspruch 1, dadurch gekennzeichnet, daß die Indolderivate ausgewählt werden unter 7-Hydroxy-2,3-dimethyl-4-methoxyindol, 7-Hydroxyindol, 5-Acetoxy-6-hydroxyindol, 6-Hydroxy-2-methyl-5-methoxyindol, 2-Carboxy-5,6-dihydroxyindol, 5,6-Di(trimethylsilyloxy)indol, 2-Methyl-4-hydroxy-5-ethoxyindol.

3. Verfahren nach Anspruch 1, dadurch gekennzeichnet, daß die Indolderivate ausgewählt werden unter 7-Hydroxy-2,3-dimethyl-4-methoxyindol, 7-Hydroxyindol, 5-Acetoxy-6-hydroxyindol und 2-Methyl-4-hydroxy-5-ethoxyindol.

4. Verfahren nach den Ansprüchen 1 bis 3, dadurch gekennzeichnet, daß das Indolderivat der Formel (I) in zwischen 0,02 und 5 Gew.-% und vorzugsweise zwischen 0,05 und 3 Gew.-% liegenden Anteilen vorhanden ist.

5. Verfahren nach einem der Ansprüche 1 bis 4, dadurch gekennzeichnet, daß das Milieu der Färbungszusammensetzung Wasser oder eine Mischung Wasser-Lösungsmittel ist, wobei das/die Lösungsmittel ausgewählt wird/werden unter Ethylalkohol, Propyl- oder Isopropylalkohol, tert.-Butylalkohol, Ethylenglykol, Monomethyl-, Monoethyl- oder Monobutylethern von Ethylenglykol, Propylenglykol, Monomethylethern von Propylenglykol und Dipropylenglykol und Methyllactat.

6. Verfahren nach einem der Ansprüche 1 bis 5, dadurch gekennzeichnet, daß die Zusammensetzung ebenfalls ein grenzflächenaktives Mittel und ein Verdickungsmittel enthält.

7. Verfahren nach einem der Ansprüche 1 bis 6, dadurch gekennzeichnet, daß die Färbungszusammensetzung zusätzlich andere Indolverbinndungen enthält.

8. Verfahren nach Anspruch 7, dadurch gekennzeichnet, daß die anderen Indolverbindungen ausgewählt werden unter 5,6-Di-hydroxyindol, 2-Methyl-5,6-dihydroxyindol, 6-Hydroxyindol und ihren entsprechenden Säureadditionssalzen.

9. Verfahren nach einem der Ansprüche 1 bis 8, dadurch gekennzeichnet, daß die Färbungszusammensetzung zusätzlich andere Farbstoffe, ausgewählt unter Direktfarbstoffen, Vorläufern von Oxidationsfarbstoffen, Kupplern, schnellen Oxidationsfarbstoffen, einschließt.

10. Verfahren nach einem der Ansprüche 1 bis 9, dadurch gekennzeichnet, daß die oxidierende Lösung ein Oxidationsmittel, ausgewählt unter Wasserstoffperoxid, Harnstoffperoxid, den aus Perkarbonaten, Perboraten von Alkalimetallen oder Ammonium ausgewählten Persalzen, enthält.

11. Verfahren nach einem der Ansprüche 1 bis 10, dadurch gekennzeichnet, daß die in der oxidierenden Lösung verwendeten alkalischen Mittel unter Ammoniak oder Alkanolaminen ausgewählt werden.

12. Verfahren nach einem der Ansprüche 1 bis 11, dadurch gekennzeichnet, daß der Anteil des Oxidationsmittels in der alkalischen, oxidierenden Zusammensetzung, welche man auf die Haare aufbringt, zwischen 1 und 10 Gew.-%, bezogen auf das Gesamtgewicht der oxidierenden, alkalischen Zusammensetzung, vorzugsweise zwischen 1 und 5 %, beträgt.

13. Färbungsmittel für Keratinfasern, insbesondere Haare, dadurch gekennzeichnet, daß es zwei Bestandteile umfaßt, wobei der erste Bestandteil (A) durch eine Färbungszusammensetzung gebildet wird, die in einem kosmetisch annehmbaren, wässrigen Milieu eine Verbindung der Formel (I), wie in einem der Ansprüche 1 bis 9 definiert, umfaßt, und der zweite Bestandteil (B) durch eine oxidierende, alkalische Lösung gebildet wird, die durch Mischen einer wässrigen Lösung eines Oxidationsmittels mit einer alkalischen Lösung, wie in einem der Ansprüche 1 und 10 bis 12 definiert, hergestellt worden ist.

14. Vorrichtung mit mehreren Abteilungen oder Färbungskit, dadurch gekennzeichnet, daß sie/es wenigstens zwei getrennte Abteilungen umfaßt, welche jeweils die in Anspruch 13 definierten Bestandteile (A) und (B), einschließen.

15. Verfahren zur Herstellung eines Färbungsmittels für Keratinfasern, insbesondere Haare, dadurch gekennzeichnet, daß man in ein kosmetisch annehmbares, wässriges Milieu, wie in einem der Ansprüche 1 und 5 bis 9 definiert, eine Verbindung der Formel (I), wie in einem der Ansprüche 1 bis 4 definiert, zum Erhalt des ersten Bestandteiles (A) eingibt, und daß man eine wässrige Lösung eines Oxidationsmittels mit einer wässrigen, alkalischen Lösung zum Erhalt einer oxidierenden, alkalischen Lösung, wie in einem der Ansprüche 1 und 10 bis 12 definiert, welche den Bestandteil (B) bildet, vermischt.